# DEMANDE DE BREVET EUROPEEN

(11) **EP 4 183 388 A1**
(43) Date de publication de la demande: **24.05.2023**
(21) Numéro de dépôt: 22207715.8
(22) Date de dépôt: 16.11.2022
(51) Int. Cl.: A61K 9/16, A61K 9/20, A61K 9/24, A61K 36/185, A61K 31/4045, A61K 8/02, A61K 8/49, A61K 8/73, A61K 8/9789, A61Q 90/00, A23L 33/10, A23L 33/105

(54) **COMPOSITION DESTINÉE À UNE ADMINISTRATION CHEZ UN SUJET HUMAIN OU ANIMAL, AVANTAGEUSEMENT PAR VOIE ORALE**

(30) Priorité: 18.11.2021 FR 2112201
(71) Demandeur: Larena, 49270 Oree D'Anjou (FR)
(72) Inventeur: DUBOURDEAUX, Michel, 03140 Taxat (FR); BARDOT, Valérie, 03140 TARGET (FR)
(74) Mandataire: Jacobacci Coralis Harle

(57) **Abrégé**

La présente invention concerne une composition destinée à une administration chez un sujet humain ou animal, avantageusement par voie orale.

Ce composition comprend au moins les composants suivants :
- un extrait de baobab,
- de l'alginate,
- une poudre imprégnée, comprenant un support végétal sur lequel est fixé un extrait de plante concentré, et
- au moins un principe actif.

## Description

### Domaine technique de l'invention

La présente invention concerne le domaine technique des compositions destinées à une administration chez un sujet humain ou animal, avantageusement par voie orale.

### Etat de la technique

Les compositions actuelles, destinées à une administration chez un sujet humain ou animal, avantageusement par voie orale, ne sont pas toujours optimales sur le plan du contrôle de la délivrance et la biodisponibilité.

En effet, pour certains principes actifs, il est utile d'obtenir deux phases de libération, ou pics de libération, à savoir une phase de libération immédiate suivi d'une phase de libération modifiée (prolongée et différée).

De telles compositions sont par exemple utiles pour l'administration de mélatonine en deux phases, de sorte à obtenir :
- une libération rapide de mélatonine, pour aider à l'endormissement, puis
- une libération progressive de mélatonine, pendant la nuit, pour améliorer la durée et la qualité du sommeil et pour limiter les réveils nocturnes.

Il existe ainsi un besoin de nouvelles compositions pouvant générer de telles phases de libération.

### Présentation de l'invention

Afin de remédier à l'inconvénient précité de l'état de la technique, la présente invention propose une composition destinée à une administration chez un sujet humain ou animal, avantageusement par voie orale.

Cette composition comprend au moins les composants suivants :
- un extrait de baobab,
- de l'alginate,
- une poudre imprégnée, comprenant un support végétal sur lequel est fixé un extrait de plante concentré, et
- au moins un principe actif.

De manière surprenante et inattendue, la composition selon l'invention permet l'obtention des deux phases de libération recherchées, à savoir une phase de libération immédiate suivi d'une phase de libération progressive.

D'autres caractéristiques non limitatives et avantageuses du produit conforme à l'invention, prises individuellement ou selon toutes les combinaisons techniquement possibles, sont les suivantes :
- le pourcentage relatif, en poids, des composants extrait de baobab / alginate / poudre imprégnée, est de 27 à 45% dudit extrait de baobab, de 3,5 à 6,5 % de ladite alginate, de 18 à 30 % de ladite poudre imprégnée ;
- ladite composition se présente sous une forme choisie parmi les comprimés, les capsules, les gélules, une poudre ; de préférence, la composition se présente sous la forme de comprimés, lesdits comprimés consistent en des comprimés multicouches superposées, par exemple des comprimés bicouches, dits encore comprimés double face, dont au moins une première couche comprend ladite association extrait de baobab, alginate, poudre imprégnée et principe actif ou des comprimés monocouche ; de préférence encore, dans le cas de comprimés bicouches, une seconde couche desdits comprimés bicouches comprend au moins les composants suivants : un extrait de baobab, de l'alginate, et au moins un principe actif, identique ou différent de ladite première couche ;
- l'extrait de baobab consiste en de la pulpe du fruit de baobab ;
- l'alginate consiste en un alginate choisi parmi l'alginate de calcium, de sodium, de potassium, de propylène glycol ;
- ladite poudre imprégnée est issue d'un procédé de fabrication comprenant les étapes successives suivantes : une étape de fourniture d'un extrait végétal concentré, ledit extrait végétal concentré étant issu d'une opération d'extraction à partir d'une matière végétale suivi d'une opération de concentration dudit extrait végétal, une étape d'imprégnation dudit extrait végétal concentré sur ledit support végétal, par exemple une étape de pulvérisation, et une étape de séchage dudit support végétal imprégné par ledit extrait végétal concentré, pour obtenir ladite poudre imprégnée ;
- le principe actif est choisi parmi les probiotiques, bactéries, levures, plantes et extraits de plantes, vitamines, minéraux et oligoéléments, substances nutritionnelles.

La présente invention concerne encore une utilisation non-thérapeutique d'une composition selon l'invention, contenant un principe actif non-thérapeutique, pour l'obtention de deux phases de libération successives par administration par voie orale :
- une phase de libération immédiate, avantageusement de 1 à 30 min et avantageusement de 10 à 20 %, en poids, dudit au moins un principe actif, et
- une phase de libération modifiée, de préférence prolongée, retardée et avantageusement à effet matriciel, avantageusement de 3 à 5 heures, et avantageusement de 30 à 50 %, en poids, dudit au moins un principe actif, de préférence en aval de l'estomac, avantageusement au niveau intestinal et/ou du colon.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée, en lien avec les exemples où :
[Fig. 1] est un graphique illustrant le profil moyen de dissolution en cumulé, montrant un pourcentage de libération en cumulé de la mélatonine en fonction du temps pour un comprimé bicouches ;
[Fig. 2] est un graphique illustrant le profil moyen de dissolution en instantané, avec un pourcentage de libération en instantané de la mélatonine en fonction du temps pour un comprimé bicouches ;
[Fig. 3] est un graphique illustrant le profil moyen de dissolution en cumulé, montrant un pourcentage de libération en cumulé de la mélatonine en fonction du temps pour un comprimé monocouche ;
[Fig. 4] est un graphique illustrant le profil moyen de dissolution en instantané, avec un pourcentage de libération en instantané de la mélatonine en fonction du temps pour un comprimé monocouche.

La présente invention concerne ainsi une composition destinée (dit encore « adaptée ») à une administration chez un sujet humain ou animal, avantageusement par voie orale.

En d'autres termes, la présente invention concerne une composition pour utilisation chez un sujet humain ou animal, avantageusement par voie orale.

Par « voie orale » ou « per os », on entend avantageusement une composition destinée à être avalée par le sujet, en vue d'une libération des principes actifs.

La composition selon l'invention peut être utilisée dans diverses applications et marchés, à savoir par exemple, avantageusement et non limitativement, nutritionnelles, alimentaires, pharmaceutiques, compléments alimentaires, dispositifs médicaux, aliments diététiques destinés à des fins médicales spéciales, cosmétiques ou nutrition animale.

La composition selon l'invention comprend au moins les composants suivants :
- un extrait de baobab,
- de l'alginate,
- une poudre imprégnée, comprenant un support végétal sur lequel est fixé un extrait de plante concentré, et
- au moins un principe actif.

De manière préférentielle, le pourcentage relatif, en poids, des composants extrait de baobab / alginate / poudre imprégnée dans la composition (éventuellement un comprimé monocouche ou multicouches comme développé par la suite), est :
- de 27 à 45% de l'extrait de baobab,
- de 3,5 à 6,5 % de l'alginate,
- de 18 à 30 % de la poudre imprégnée.

De préférence, les pourcentages relatifs sont exprimés par rapport au poids total de la composition.

### Extrait de baobab

Par « extrait de baobab », on entend en particulier la pulpe du fruit de baobab (*Adansonia digitata* L.).

Selon un mode de réalisation préféré de l'invention, la pulpe de fruit de baobab est utilisée sous forme d'une poudre de pulpe de fruit de baobab (de préférence se présentant sous forme de particules micronisées).

Une telle poudre est avantageusement obtenue à partir de la pulpe du fruit du baobab, de préférence par séparation mécanique (en particulier par séparation mécanique de la pulpe et des graines) et pulvérisation (avantageusement par micronisation de la pulpe), naturellement déshydratée à l'intérieur du fruit.

De préférence, la poudre de pulpe de fruit de baobab se présente sous forme de particules de taille micronique, telles que des particules micronisées.

De préférence lesdites particules de taille micronique ont une granulométrie inférieure ou égale à 1000 µm, préférablement inférieure à 1000 µm. Avantageusement, lesdites particules ont une granulométrie inférieure à 900 µm, et de manière préféré inférieure à 800 µm.

A cet égard, les caractéristiques granulométriques de la poudre de pulpe de fruit de baobab sont avantageusement les suivantes :
- une taille inférieure, ou égale, à 50 µm représentant de préférence de 70 à 80%,
- une taille inférieure, ou égale, à 100 µm représentant de préférence de 90 à 100%.

De manière classique en soi, les caractéristiques granulométriques sont avantageusement issues d'une technique granulométrique par tamisage.

Le pourcentage consiste avantageusement en un pourcentage en masse.

Le pourcentage en masse en question représente avantageusement la partie de la poudre imprégnée qui reste sur le tamis, dit encore « refus » ou « refus par tamis ».

De telles valeurs peuvent être mesurées selon un protocole décrit par exemple dans le document suivant : Pharmacopée Européenne 9.0, « 2.9.38. Distribution granulométrique par tamisage analytique » ou Pharmacopée Européenne 9.0, « 2.9.35. Finesse des poudres ».

### Alginate

L'alginate assure avantageusement un rôle de gélifiant, avantageusement en présence d'ions divalents comme le calcium, qui crée des ponts entre les chaînes des polymères.

L'alginate est avantageusement extrait de la paroi d'algues brunes, les laminaires.

L'alginate consiste avantageusement en de l'alginate de calcium, de sodium, de potassium ou de propylène glycol.

La viscosité de l'alginate est de préférence comprise de 350 à 550 mPa.s (cps).

### Poudre imprégnée

La poudre imprégnée comprend ainsi un support végétal sur lequel est fixé un extrait de plante concentré.

De préférence, le support végétal et l'extrait de plante concentré sont issus de mélisse officinale (*Melissa officinalis* L.).

Sans être limitatif, le support végétal et l'extrait de plante peuvent encore être issus d'une plante choisie parmi l'une des plantes suivantes : passiflore, valériane, eschscholtzia, plantain, rhodiola, échinacée, artichaut, marc de raisin, thé vert, vigne rouge, aubépine, romarin, éleuthérocoque, cassis, maté, reine des près, prêle, houblon, radis noir, chardon marie, curcuma, desmodium, gingembre, ginseng, gingko, griffonia, harpagophytum, noyer, réglisse, etc.

En pratique, la poudre imprégnée consiste avantageusement en une poudre imprégnée qui est avantageusement issue d'un procédé de fabrication comprenant les étapes successives suivantes :
- une étape de fourniture d'un extrait végétal concentré, ledit extrait végétal concentré étant issu d'une opération d'extraction à partir d'une matière végétale suivi d'une opération de concentration dudit extrait végétal,
- une étape d'imprégnation dudit extrait végétal concentré sur ledit support végétal, par exemple une étape de pulvérisation, et
- une étape de séchage dudit support végétal imprégné par ledit extrait végétal concentré, pour obtenir ladite poudre imprégnée.

De manière non limitative, un tel procédé de fabrication est par exemple décrit dans le document EP-2 080 436 « Procédé de préparation d'extraits végétaux permettant l'obtention d'une nouvelle forme galénique ».

En substance, l'opération d'extraction comprend avantageusement les étapes suivantes :
- une étape de contact entre la matière biologique d'intérêt et un solvant,
- au moins une étape d'extraction, proprement dite, d'au moins une partie des principes actifs présents dans ladite matière biologique d'intérêt (par migration dans le solvant).

L'opération de concentration consiste alors avantageusement à concentrer les principes actifs par élimination d'au moins une partie du solvant d'extraction.

La concentration est avantageusement d'un facteur de concentration d'au moins 5, de préférence d'un facteur de concentration de 8 à 12.

Selon une forme de réalisation préférée, l'extrait biologique concentré consiste en un extrait végétal concentré. L'extrait biologique concentré est issu d'une opération d'extraction à partir d'une matière végétale, suivie d'une opération de concentration dudit extrait biologique.

Dans ce cadre, la matière végétale consiste avantageusement en une matière végétale fraîche ou sèche, avantageusement sur une partie de plante ou une plante entière.

L'opération d'extraction comprend avantageusement une mise en contact de la matière végétale avec un solvant, dans lequel va avoir lieu l'extraction des principes actifs.

Ensuite, par exemple, l'étape d'imprégnation consiste en une étape de pulvérisation, de préférence une étape de pulvérisation de l'extrait biologique concentré sur le support biologique.

De préférence, le support biologique se présente sous la forme d'une poudre.

Selon une forme de réalisation préférée, le support biologique consiste en un support végétal.

Dans ce cadre, le support végétal consiste avantageusement en une matière végétale sèche, sous forme de poudre, avantageusement une partie de plante ou une plante entière.

De préférence, le support biologique est de même nature que la matière biologique utilisée pour l'obtention de l'extrait biologique concentré.

En ce sens, l'extrait biologique concentré et le support biologique sont avantageusement un extrait végétal concentré et un support végétal.

L'étape de séchage correspond à une phase de séchage en présence du support biologique sur lequel a été déposé l'extrait biologique concentré au cours de l'opération d'imprégnation.

Cette étape de séchage a pour but essentiel d'entraîner l'évaporation du solvant apporté par l'extrait biologique concentré.

Cette étape de séchage est avantageusement mise en oeuvre jusqu'au séchage complet de la poudre imprégnée.

De manière simultanée, ce séchage permet la fixation des principes actifs (apportés par l'extrait biologique concentré) sur le support biologique.

A l'issue du procédé, la poudre imprégnée se présente donc sous une forme sèche.

De préférence, la poudre imprégnée peut présenter les caractéristiques granulométriques suivantes :
- une taille inférieure, ou égale, à 50 µm représentant au maximum 25%, de préférence de 10 à 25%,
- une taille inférieure, ou égale, à 100 µm représentant au maximum 50%, de préférence de 25 à 50%, et
- une taille inférieure, ou égale, à 500 µm représentant au minimum 90%, de préférence de 95 à 100%.

De préférence, la poudre imprégnée présente encore :
- une masse volumique apparente, ou plus précisément une masse volumique apparente sans tassement, allant de 0,3 à 0,8 g/cm³, et
- une masse volumique tassée, ou masse volumique apparente après tassement, allant de 0,4 à 0,9 g/cm³.

De manière classique en soi, les caractéristiques granulométriques sont avantageusement issues d'une technique granulométrique par tamisage.

Le pourcentage en masse en question représente avantageusement la partie de la poudre imprégnée qui reste sur le tamis, dit encore « refus » ou « refus par tamis ».

De telles valeurs peuvent être mesurées selon un protocole décrit par exemple dans le document suivant : Pharmacopée Européenne 9.0, « 2.9.38. Distribution granulométrique par tamisage analytique » ou Pharmacopée Européenne 9.0, « 2.9.35. Finesse des poudres ».

### Principe actif

Par « principes actifs », on englobe avantageusement les principes actifs thérapeutiques et les principes actifs non-thérapeutiques.

On englobe avantageusement les probiotiques, bactéries, levures, plantes et extraits de plantes, vitamines, minéraux et oligoéléments, substances nutritionnelles, etc.

Plus généralement, le principe actif peut être choisi parmi un agent pharmaceutique, un agent vétérinaire, un complément nutritionnel, un agent agricole, un ingrédient cosmétique, un colorant et un aliment.

### Forme de la composition

La composition selon l'invention se présente avantageusement sous une forme choisie parmi :
- les comprimés,
- les capsules,
- les gélules,
- une poudre.

Ces formes, avec leurs procédés de fabrication respectifs, sont par exemple enseignés dans les documents Phi41, Pharmacotechnie industrielle, Edition IMT, 07/2016 ou Pharmacie galénique: Bonnes pratiques de fabrication des médicaments, Edition Elsevier Masson, 10e édition (19 octobre 2016).

Selon une forme de réalisation préférée, la composition se présente sous la forme de comprimés.

Les comprimés consistent avantageusement en :
- des comprimés multicouches superposées, par exemple des comprimés bicouches qui sont dits encore comprimés double face, ou
- des comprimés monocouches.

De tels comprimés comportent au moins une première couche comprend ladite association :
- extrait de baobab,
- alginate,
- poudre imprégnée et
- principe actif.

Dans cette première couche, le pourcentage relatif, en poids, des composants extrait de baobab / alginate / poudre imprégnée (avantageusement par rapport au poids total de la composition), est avantageusement :
- de 5 à 10% dudit extrait de baobab,
- de 0,5 à 5 % de ladite alginate,
- de 18 à 30 % de ladite poudre imprégnée.

De préférence, les comprimés consistent en des comprimés bicouches.

Dans ce cas, une seconde couche de ces comprimés bicouches comprend au moins les composants suivants :
- un extrait de baobab,
- de l'alginate, et
- au moins un principe actif, identique ou différent de ladite première couche.

Dans cette seconde couche, le pourcentage relatif, en poids, des composants extrait de baobab / alginate (avantageusement par rapport au poids total de la composition), est avantageusement de :
- de 25 à 35% dudit extrait de baobab,
- de 3 à 6 % de ladite alginate.

De préférence, chaque couche représente au moins 40%, en masse, du comprimé bicouches.

Encore de préférence, la quantité de principe actif dans la seconde couche est supérieure à la quantité de principe actif dans la première couche.

De manière alternative, les comprimés consistent en des comprimés monocouches.

Encore de manière générale, le comprimé présente avantageusement les caractéristiques suivantes :
- une uniformité de masse allant de 700 à 900 mg,
- une épaisseur allant de 5 à 7 mm,
- une dureté allant de 150 à 300 N,
- une friabilité inférieure à 1%.

### Utilisation de l'invention

La présente invention forme avantageusement un complexe naturel ayant une fonction de protection des principes actifs et agissant ainsi sur le contrôle de la délivrance et la biodisponibilité.

La composition selon l'invention est en particulier intéressante dans une utilisation pour l'obtention de deux phases de libération successives d'un principe actif (par exemple la mélatonine), par administration par voie orale :
- une phase de libération immédiate, avantageusement de 1 à 30 min (après administration) et avantageusement de 10 à 20 %, en poids, dudit au moins un principe actif, et
- une phase de libération modifiée, de préférence prolongée, retardée et avantageusement à effet matriciel, avantageusement de 3 à 5 heures (après administration), et avantageusement de 30 à 50 %, en poids, dudit au moins un principe actif, de préférence en aval de l'estomac, avantageusement au niveau intestinal et/ou du colon.

En d'autres termes, la phase de libération immédiate est avantageusement active de 1 à 30 min après administration ; et la phase de libération modifiée, à distance de l'administration, est avantageusement active de 3 à 5 heures après administration.

En d'autres termes, les deux phases de libération successives sont avantageusement séparées temporellement et à distance dans le temps, avantageusement sous la forme de deux pics.

Le pourcentage exprime la quantité en poids par rapport au poids total, dudit au moins un principe actif, qui est libéré au cours de chaque phase de libération.

Bien entendu, diverses autres modifications peuvent être apportées à l'invention dans le cadre des revendications annexées.

Bien entendu encore, les différentes caractéristiques, variantes et formes de réalisation de l'invention peuvent être associées les unes avec les autres selon diverses combinaisons dans la mesure où elles ne sont pas incompatibles ou exclusives les unes des autres.

### Exemples

Des études de la dissolution ont été mises en œuvre sur la base de la monographie de la Pharmacopée Européenne « 5.17.1. Recommandations relatives à l'essai de dissolution » à pH 1,2 et 6,8.

### Exemple 1 - Comprimés bicouches

Une première étude de dissolution a été mise en œuvre à partir de comprimés bicouches dont la formule est détaillée ci-dessous :

**[Tableau 1]**

| Dénomination | Dosage (%) |
|---|---|
| Couche 1 | |
| MELATONINE 99% | 0,04 à 0,05 |
| Poudre imprégnée - MELISSE | 20 à 25 |
| Pulpe de baobab | 5 à 7 |
| ALGINATE DE SODIUM | 0,5 à 1,5 |
| Excipients | q.s.p. |
| TOTAL COUCHE 1 | 40 à 50 |
| | |
| Couche 2 | |
| MELATONINE 99% | 0,1 à 0,2 |
| Pulpe de baobab | 25 à 35 |
| ALGINATE DE SODIUM | 4 à 5 |
| Excipients | q.s.p. |
| TOTAL COUCHE 2 | 50 à 60 |
| | |
| TOTAL dosage : | 100,000 |

Le comprimé bicouches présente les caractéristiques suivantes :
- uniformité de masse : 700 à 900 mg,
- épaisseur : 5 à 7 mm,
- dureté : 150 à 300 N,
- friabilité : inférieure à 1%,
- temps de désagrégation : environ 1 heure.

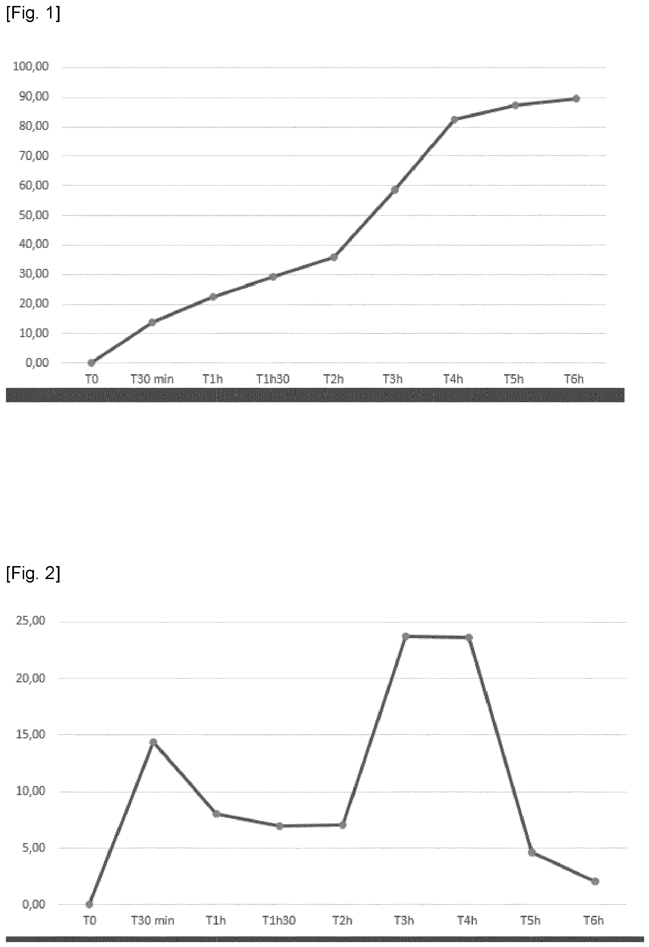

Les profils cumulés moyens de dissolution et les vitesses instantanées moyennes de dissolution sont représentés, respectives, dans les figures 1 et 2.

De manière surprenante et inattendue, le profil de dissolution présente deux pics :
- un premier pic à 30 minutes avec une libération d'environ de 10 à 20 %, en poids, de préférence 15%, du principe actif (mélatonine) et
- un second pic entre 3 et 4 heures avec une libération de 30 à 50%, en poids, de préférence à 45%, du principe actif (mélatonine).

### Exemple 2 - Comprimés monocouches

Une seconde étude de dissolution a été mise en œuvre à partir de comprimés monocouches dont la formule est détaillée ci-dessous :

**[Tableau 2]**

| Dénomination | Dosage (%) |
|---|---|
| MELATONINE 99% | 0,2 à 0,3 |
| Poudre imprégnée - MELISSE | 20 à 30 |
| Pulpe de baobab | 30 à 40 |
| ALGINATE DE SODIUM | 5 à 6 |
| Excipients | q.s.p. |
| TOTAL dosage | 100,000 |

Le comprimé monocouche présente les caractéristiques suivantes :
- uniformité de masse : 700 à 900 mg,
- épaisseur : 5 à 7 mm,
- dureté : 150 à 300 N,
- friabilité : inférieure à 1%,
- temps de désagrégation : entre 45 minutes et 1 heure.

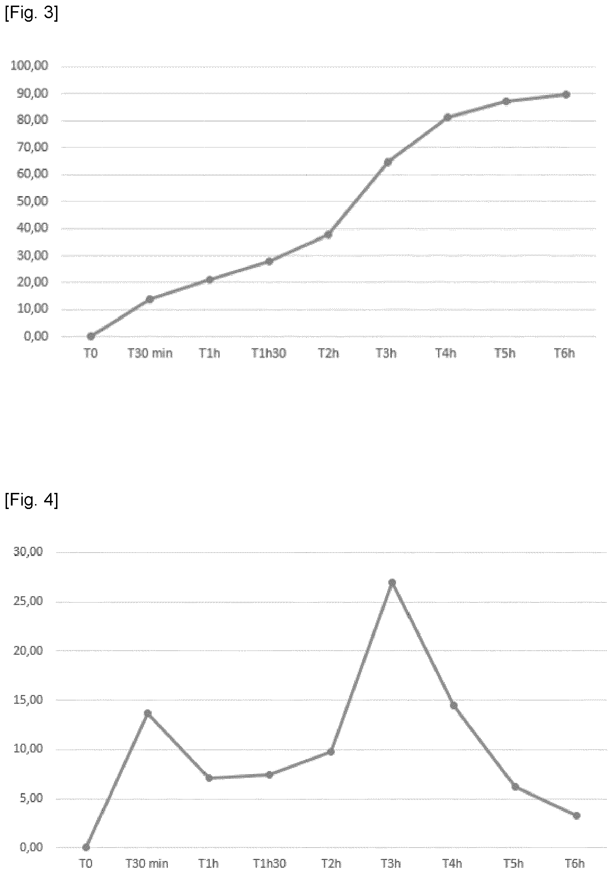

Les profils cumulés moyens de dissolution et les vitesses instantanées moyennes de dissolution sont représentés, respectives, dans les figures 3 et 4 (pour moyenne de 7 bols).

De manière surprenante et inattendu, le profil de dissolution présente encore deux pics :
- un premier pic à 30 minutes avec une libération d'environ de 10 à 20 %, en poids, de préférence 15%, du principe actif (mélatonine) et
- un second pic entre 3 et 4 heures avec une libération de 30 à 50%, en poids, de préférence à 45%, du principe actif (mélatonine).

## Revendications

1. Composition destinée à une administration chez un sujet humain ou animal, avantageusement par voie orale, **caractérisée en ce qu'**elle comprend au moins les composants suivants :
- un extrait de baobab,
- de l'alginate,
- une poudre imprégnée, comprenant un support végétal sur lequel est fixé un extrait de plante concentré, et
- au moins un principe actif.

2. Composition selon la revendication 1, **caractérisée en ce que** le pourcentage relatif, en poids, des composants extrait de baobab / alginate / poudre imprégnée, est :
- de 27 à 45% dudit extrait de baobab,
- de 3,5 à 6,5 % de ladite alginate,
- de 18 à 30 % de ladite poudre imprégnée.

3. Composition selon l'une quelconque des revendications 1 ou 2, **caractérisée en ce que** ladite composition se présente sous une forme choisie parmi :
- les comprimés,
- les capsules,
- les gélules,
- une poudre.

4. Composition selon la revendication 3, dans laquelle la composition se présente sous la forme de comprimés, **caractérisée en ce que** lesdits comprimés consistent en :
- des comprimés multicouches superposées, par exemple des comprimés bicouches, dits encore comprimés double face, dont au moins une première couche comprend ladite association extrait de baobab, alginate, poudre imprégnée et principe actif, ou
- des comprimés monocouches.

5. Composition selon la revendication 4, **caractérisée en ce que**, dans le cas de comprimés bicouches, une seconde couche desdits comprimés bicouches comprend au moins les composants suivants :
- un extrait de baobab,
- de l'alginate, et
- au moins un principe actif, identique ou différent de ladite première couche.

6. Composition selon l'une quelconque des revendications 1 à 5, **caractérisée en ce que** l'extrait de baobab consiste en de la pulpe du fruit de baobab.

7. Composition selon l'une quelconque des revendications 1 à 6, **caractérisée en ce que** l'alginate consiste en un alginate choisi parmi de l'alginate de calcium, de sodium, de potassium ou de propylène glycol.

8. Composition selon l'une quelconque des revendications 1 à 7, **caractérisée en ce que** ladite poudre imprégnée est issue d'un procédé de fabrication comprenant les étapes successives suivantes :
- une étape de fourniture d'un extrait végétal concentré, ledit extrait végétal concentré étant issu d'une opération d'extraction à partir d'une matière végétale suivi d'une opération de concentration dudit extrait végétal,
- une étape d'imprégnation dudit extrait végétal concentré sur ledit support végétal, par exemple une étape de pulvérisation, et
- une étape de séchage dudit support végétal imprégné par ledit extrait végétal concentré, pour obtenir ladite poudre imprégnée.

9. Composition selon l'une quelconque des revendications 1 à 8, **caractérisée en ce que** le principe actif est choisi parmi les probiotiques, bactéries, levures, plantes et extraits de plantes, vitamines, minéraux et oligoéléments, substances nutritionnelles.

10. Utilisation d'une composition selon l'une quelconque des revendications 1 à 9, contenant un principe actif non-thérapeutique, pour l'obtention de deux phases de libération successives par administration par voie orale :
- une phase de libération immédiate, avantageusement de 1 à 30 min et avantageusement de 10 à 20 %, en poids, dudit au moins un principe actif, et
- une phase de libération modifiée, de préférence prolongée, avantageusement de 3 à 5 heures, et avantageusement de 30 à 50 % en poids dudit au moins un principe actif, de préférence en aval de l'estomac, avantageusement au niveau intestinal et/ou du colon.
